(19) Europäisches Patentamt / European Patent Office / Office européen des brevets

(11) **EP 4 125 553 B1**

(12) **EUROPEAN PATENT SPECIFICATION**

(45) Date of publication and mention
of the grant of the patent:
**13.09.2023 Bulletin 2023/37**

(21) Application number: **21713437.8**

(22) Date of filing: **26.03.2021**

(51) International Patent Classification (IPC):
**A61B 5/00** *(2006.01)*  **H04M 1/72463** *(2021.01)*
**A61B 5/05** *(2021.01)*  **A61B 5/0522** *(2021.01)*
**A61B 5/024** *(2006.01)*  **A61B 5/08** *(2006.01)*

(52) Cooperative Patent Classification (CPC):
**A61B 5/6898; A61B 5/0015; A61B 5/05;**
**A61B 5/0522; H04M 1/72463;** A61B 5/002;
A61B 5/024; A61B 5/0816

(86) International application number:
**PCT/EP2021/057860**

(87) International publication number:
**WO 2021/198045 (07.10.2021 Gazette 2021/40)**

(54) **CONTROLLER AND METHOD FOR SWITCHING BETWEEN SENSING AND NON-SENSING MODES IN THE CONTEXT OF A PORTABLE HANDHELD DEVICE ASSOCIATED WITH THE INDUCTIVE SENSING.**

STEUERGERÄT UND VERFAHREN ZUM UMSCHALTEN ZWISCHEN ABTAST- UND NICHTABTASTMODUS IM ZUSAMMENHANG MIT EINEM TRAGBAREN HANDGERÄT, DAS MIT DER INDUKTIVEN ABTASTUNG VERBUNDEN IST.

CONTRÔLEUR ET MÉTHODE DE COMMUTATION ENTRE LES MODES DE DÉTECTION ET DE NON-DÉTECTION DANS LE CONTEXTE D'UN DISPOSITIF PORTABLE ASSOCIÉ À LA DÉTECTION INDUCTIVE.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2020 EP 20167495**

(43) Date of publication of application:
**08.02.2023 Bulletin 2023/06**

(73) Proprietor: **Koninklijke Philips N.V.**
**5656 AG Eindhoven (NL)**

(72) Inventors:
• **PEETERS, Wouter, Herman**
  **5656 AE Eindhoven (NL)**
• **STEUNEBRINK, Tim, Patrick**
  **5656 AE Eindhoven (NL)**
• **HARBERTS, Dirk, Willem**
  **5656 AE Eindhoven (NL)**

• **DOODEMAN, Gerardus, Johannes, Nicolaas**
  **5656 AE Eindhoven (NL)**
• **VERNOOIJ, Carlijn, Andrea**
  **5656 AE Eindhoven (NL)**

(74) Representative: **Philips Intellectual Property & Standards**
**High Tech Campus 52**
**5656 AG Eindhoven (NL)**

(56) References cited:
EP-A1- 2 418 780      EP-A1- 3 583 896
US-A1- 2018 206 758   US-A1- 2019 343 417

• **HYE RAN KOO ET AL: "The Effect of Textile-Based Inductive Coil Sensor Positions for Heart Rate Monitoring", JOURNAL OF MEDICAL SYSTEMS, vol. 38, no. 2, 31 January 2014 (2014-01-31), XP055145252, ISSN: 0148-5598, DOI: 10.1007/s10916-013-0002-0**

**Description**

FIELD OF THE INVENTION

This invention relates to a controller and method in the field of inductive sensing, in particular to a means for switching between sensing and non-sensing modes in the context of a portable handheld device associated with the inductive sensing.

BACKGROUND OF THE INVENTION

Worldwide, at all levels of healthcare, there is a need to do basic patient triaging. Quick, initial triaging is important in order to categorize waiting patients into an order of priority based on the urgency of their medical condition. For example, many areas in the world have overcrowded primary healthcare facilities, where waiting patients are in danger of deterioration while waiting in queues for examination. In low-resource countries, there is a need to screen patients for hospital referral by relatively low-skilled personnel. Even in high-resource countries or hospitals, there is a need to do basic patient triaging, for example in the emergence room (ER).

In developed countries, there is also an increasing need for regular spot-checks and triaging in lower-acuity care settings, due to a trend to "push back" patients to lower acuity care to save costs (e.g. skilled nursing facilities, a patient's home, remote GP visits).

There is furthermore a need for a quick and simple means for detecting the presence of cardiac arrest, preferably which can be used by both clinicians and non-expert operators.

Two basic vital-sign measurements that can provide indication of a wide range of different pathologies are pulse rate and breathing rate. These can give an indication of abnormalities in condition and also a danger of deterioration.

In a standard hospital settings, measurement of heart rate and breathing rate is done with two separate devices, and these are typically large units, for example carried on trollies. Thus, the patient must be brought to a location, e.g. a triaging bed, at which both devices are located and plugged in. In addition, standard devices for measuring heart rate and breathing rate require direct-contact sensors, e.g. PPG finger clip sensor for heart rate, meaning that these sensors have to be cleaned between patients, or contact portions of the sensors completely replaced, to avoid cross-contamination.

In view of the above, it would be of value to have a simple, mobile, contactless and readily accessible device for performing assessment of vital signs of a patient, preferably including at least pulse rate and breathing rate.

EP 3584896 describes a marker device and a tracking system for tracking the marker device.

SUMMARY OF THE INVENTION

It has been recognized by the inventors that the increasing ubiquity of smartphones makes use of these devices an attractive approach. Typically individuals already carry smartphones on their person for internet access, email, telephone communication and a variety of other purposes. It would therefore be very efficient to also introduce vital sign measurement functionality into these devices, since this would avoid duplication. It would also mean the measurement could be performed by low-skilled persons, and the measurement device would be always on hand. In addition, a smartphone already has integrated functionality for transmitting or communicating collected data, and for making emergency phone calls or triggering emergency alarms if this is needed.

Integration of medical sensing technology into mobile phones has been suggested before, but it has not gained widespread adoption for a number of reasons. Most particularly, a problem with smartphones is that they emit electromagnetic radiation at a higher level than is permitted by current regulations for a medical device. For example, implanted medical devices are not designed to be operational proximal to a working mobile phone. For example, a pacemaker may fail in such circumstances. A second major problem is that current solutions require contact between the mobile phone and the patient. For example, pulse and breathing rates can be measured using the camera of a mobile phone in combination with an LED, the two forming a contact photoplethysmography (PPG) sensor. Other solutions are complex or unreliable, for example camera-based methods for measuring vital signs can require high levels of lighting which are not always available, and also require the subject is very still and that the camera is held very stable. In a hospital triaging situation, these conditions are not always possible.

An improved approach to utilizing portable handheld devices having electromagnetic transmission functions for performing medical sensing of vital signs, able to overcome one or more of the above problems, would therefore be of value.

The invention is defined by the claims.

According to examples in accordance with an aspect of the disclosure there is provided a controller,

the controller comprising an input/output for receiving and transmitting data when the controller is communicatively coupled with an inductive sensing circuit and a portable handheld device, the portable handheld device having electromagnetic emitting functions, and

the controller operable to implement a switching function for switching between two modes:

a first mode in which the controller is configured to communicate with the portable handheld device to cause deactivation of at least a portion of electromagnetic emissions of the device, and to concurrently communicate with the inductive sensing circuit to cause activation of the inductive sensing circuit; and

a second mode in which the controller is configured to communicate with the portable handheld device to cause activation of said at least portion of electromagnetic emissions of the device, and to concurrently cause deactivation of the inductive sensing circuit,

wherein, in the first mode, the controller is configured to cause the portable handheld device to activate a power supply from the portable handheld device to the inductive sensing circuit or alternatively to cause the inductive sensing circuit to activate a signal generator in the inductive sensing circuit to trigger supply of a drive signal to an antenna in the inductive sensing circuit, and wherein, in the second mode, the controller is configured to cause the portable handheld device to deactivate said power supply from the portable handheld device to the inductive sensing circuit.

Preferably, the inductive sensing circuit is arranged in the first mode to draw a power supply from the portable handheld device. In this way, the inductive sensing circuit is supported or supplied by the portable handheld device and is used in association with the portable handheld device. Various relative physical and functional arrangements of the two are possible and will be discussed further below.

Preferably, the inductive sensing circuit is configured to be electrically and/or physically coupled with the portable handheld device in use.

An inductive sensing circuit means a circuit for example comprising a resonator circuit with an antenna and preferably a signal generator and configured to be driven at resonance to generate electromagnetic excitation signals for application to the body. In inductive sensing, these excitation fields induce eddy currents in the tissue of the body, and these in turn generate secondary electromagnetic emissions which interact with the primary excitation signals to alter the electromagnetic oscillations experienced at the antenna of the inductive circuit. This causes changes in electrical properties of the current in the resonator circuit, such as the resonant frequency or amplitude of the current, and by measuring these changes over time, a signal representative of tissue movements (e.g. heart or lung movements) can be derived. These signals can be used to derive measures of vital signs such as heart rate and breathing rate.

Inductive sensing uses electromagnetic emissions at a much lower power than typical emissions of a mobile phone or other mobile communication devices. They are therefore within the range of permitted emission levels for medical devices.

Ideally, such an inductive sensing circuit would be integrated in, or connected to, a portable handheld device such as a mobile phone, so that the mobile phone can for example power and optionally control and co-ordinate sensing functionality of the circuit, e.g. via an app. However, as discussed above, use of devices with levels of electromagnetic (EM) emissions as high as those of mobile portable devices are not suitable in medical contexts.

Embodiments of the present invention provide a solution to this problem by proposing effectively a switching mechanism by which an apparatus comprising a portable handheld device having electromagnetic transmission functionality and an inductive sensing circuit can be switched between medical sensing modes (first mode) and mobile communication modes (second mode). In the first mode, the proposed controller concurrently controls the electromagnetic emissions of the handheld portable device to cease or at least to be limited or restricted in a defined way (for example ceasing emissions within a particular frequency range or above a particular power, or switching off a defined subset of radio transmitters of the portable handheld device), and to activate power to the inductive sensing circuit, or to control the inductive sensing circuit to begin being driven with a drive signal to cause it to resonate. In the second mode, these two actions are reversed: the inductive sensing circuit is deactivated, and electromagnetic transmission functionality of the portable handheld device is fully activated. These two modes can be toggled between to switch between medical sensing functionality, and normal functionality of the portable handheld device.

Thus, embodiments of the present invention provide a means for automatically disabling potentially harmful electromagnetic emissions of a portable handheld device whenever medical sensing functions are to be performed with the inductive sensing circuit. By controlling the inductive sensing circuit and electromagnetic emissions of the apparatus simultaneously, this relieves user the burden of manually disabling radio transmissions of the mobile device whenever they need to perform inductive sensing, and avoids potentially forgetting to do this. This, among other things, enables such an apparatus to be compliant with regulations for medical devices, since it enables such an apparatus to guarantee medical sensing will never be performed without harmful electromagnetic transmissions being switched off.

The controller is an electronic controller. It may be a controller or control arrangement or control unit or control module or processor arrangement in different examples. It may comprise one or more processors or controllers.

The controller may be arranged to receive control commands, and to implement switching between the two modes responsive to receipt of pre-defined control commands. It may be arranged in use to receive a control command from the portable handheld device for example or from an external device.

For example, the control command might be generated upon activation of a particular operation mode by a user on the user interface of the portable handheld device.

The controller may be arranged to detect a change of state of the portable handheld device, and to implement switching responsive to detection of this change of state.

The controller can be a control unit or module for instance. This may be or may comprise one or more processors or a processing arrangement.

The controller may comprise a single controller or processing unit, or may be distributed between different controllers and/or different devices. For instance, the controller could be facilitated jointly by a processor of the portable device and a processor of a secondary unit. Deactivation/activation of EM emissions could be controlled by the portable device processor, and activation/deactivation of the sensing circuit could be controlled by the secondary sensor unit processor. The two might exchange signals co-operatively so as to together implement the switching between the two different modes.

In one or more embodiments, the first mode may comprise causing deactivation of one or more electromagnetic transmitters of the portable device.

The one or more transmitters which are deactivated may be a defined subset of the total set of transmitters that the mobile device comprises. For example, these may be transmitters which emit in a frequency or power range which is outside of regulations for medical devices.

In accordance with one or more embodiments, the first mode may comprise causing cessation of emission in one or more electromagnetic frequency bands/ranges, or may comprise causing cessation of any electromagnetic emissions above a defined threshold power.

In accordance with one or more embodiments, the controller may be further configured to receive an inductive sensing signal input from the inductive sensing circuit and to derive from the signal one or more physiological parameters, for example heart rate and/or breathing rate.

In accordance with one or more embodiments, the controller may be configured to implement switching between the two modes responsive to receipt of one or more pre-defined control commands, and preferably wherein the controller is arranged to receive said control commands from the portable handheld device.

The control commands may in certain examples be received (directly or indirectly) from a hardware switch of the portable device, or from a software-triggered command.

According to one or more embodiments, in the first mode, the controller may be configured to cause the portable handheld device to activate a power supply from the portable device to the sensing circuit. In the second mode, the controller may be configured to cause the portable handheld device to deactivate said power supply from the portable handheld device to the sensing circuit.

Examples in accordance with a further aspect of the invention also provide an inductive sensing assembly comprising:

an inductive sensing circuit for sensing electromagnetic signals returned from a body responsive to application of electromagnetic excitation signals to said body,
the inductive sensing circuit comprising a resonator circuit comprising a loop antenna, the resonator circuit for generating said excitation signals when driven with a drive signal,
a controller in accordance with any example or embodiment outlined above or described below or in accordance with any claim of the present application, operatively coupled with the inductive sensing circuit; and
a carrier, the inductive sensing circuit and controller being mounted to the carrier.

The assembly may for example be a chip.

Hence, in this aspect, there is provided an integrated chip or circuit assembly which comprises both the controller and the inductive sensing circuit for performing inductive sensing functionality. In different embodiments, the assembly could be integrated directly in a portable handheld device, for example a mobile communications device, for example a mobile telephone (smartphone) or tablet computer. Thus, the principle of the present invention could be implemented with a dedicated chip either installed in the portable handheld device or alternatively in a separate unit, for instance to which the portable handheld device is operatively coupled. The separate unit could for example comprise a sleeve article configured for being removably wrapped around the portable handheld device, the assembly (e.g. chip) being integrated in the sleeve article.

The carrier may be, or may comprise, a substrate. The substrate for example be or comprise a circuit board, e.g. a PCB.

The inductive sensing circuit may comprise a signal generator for generating a drive signal suitable for driving said antenna to generate said electromagnetic excitation signals.

For example, the signal generator may generate an alternating drive signal. For example the signal generator may be an oscillator component. For example, the signal generator may drive the antenna to resonate, for example by driving it with a signal having a frequency matching the resonance frequency of the resonator circuit.

The inductive sensing circuit may comprise a signal sensing/pick-up means for detecting signals returned from the body based on detecting variations in one or more electrical characteristics of the resonator circuit. The electrical characteristics may for example include resonance frequency and/or amplitude of the resonator circuit.

Examples in accordance with a further aspect of the invention also provide an apparatus for inductive sensing, the apparatus comprising:

> a portable handheld device having electromagnetic emitting functions, and
> a controller in accordance with any example or embodiment outlined above or described below, or in accordance with any claim of this application.

In different implementations, the controller may be implemented by an integrated (native or local) controller or processor of the portable handheld unit, or the apparatus may be provided with a dedicated controller, e.g. dedicated controller or processor, for implementing the controller. In the first case, the control functions of the controller may be implemented for example by an app or other computer code means installed on the portable device (e.g. installed on the local / native controller or processor of the device). In the second case, the dedicated controller or processor is configured to implement the control functions of the controller. For example, it may have computer code means installed or stored on it and be configured to execute this code.

The apparatus may further comprise an inductive sensing circuit, operatively coupled with the controller, the inductive sensing circuit for sensing electromagnetic signals returned from a body responsive to application of electromagnetic excitation signals to said body. The inductive sensing circuit may comprise for example a resonator circuit comprising a loop antenna, the resonator circuit for generating said excitation signals when driven with a drive signal.

The inductive sensing circuit may be arranged to draw a power supply from the portable handheld device for powering the circuit when in the first mode.

In the first mode, the controller may be configured to cause the portable handheld device to activate a power supply from the portable device to the sensing circuit.

In the second mode, the controller is configured to cause the portable handheld device to deactivate said power supply from the portable device to the sensing circuit.

There are different options for the relative spatial configuration of the various components.

For example, in one or more embodiments, the inductive sensing circuit may be integrated in the portable handheld unit, for example as part of a dedicate chip or circuit assembly.

The chip or assembly may or may not include the controller. The controller could be separate or could be part of the assembly.

In alternative examples, the apparatus may include a secondary unit, the inductive sensing circuit being integrated in the secondary unit, and the portable handheld unit being operatively coupled with the secondary unit.

The secondary unit could for example be a peripheral or auxiliary sensor unit containing the inductive circuit arrangement. The secondary unit is separate from the portable handheld unit.

In some examples for instance, the secondary unit may comprise a sleeve article configured for being removably wrapped around the portable handheld device, the inductive sensing circuit being integrated in the sleeve article.

In accordance with one or more embodiments, the apparatus may comprise an inductive sensing assembly in accordance with any example or embodiment outlined above or described below, or in accordance with any claim of this application. In this case, the controller and inductive sensing circuit referred to above are provided by said inductive sensing assembly. The inductive sensing assembly is arranged operatively coupled with the portable handheld unit in this example.

The inductive sensing assembly may be operatively coupled with e.g. a controller or processor of the portable handheld unit. For example, the sensing assembly may take the form of a chip, so that in this example the portable handheld device is provided with a dedicated inductive sensing chip which includes both the controller and the inductive sensing circuit.

The inductive sensing assembly may be integrated in the portable handheld unit.

An alternative arrangement might be for example to provide the sensing circuit on a dedicated chip, with the controller being separate. The controller could be provided by its own dedicated chip, or may be facilitated or implemented by a local/native controller or processor of the portable handheld device.

As mentioned above, the apparatus may include a secondary unit. The inductive sensing assembly may in some examples be integrated in the secondary unit, and the portable handheld unit being operatively coupled with the secondary unit, e.g. with the sensing assembly of the secondary unit.

The secondary unit could for example be a peripheral or auxiliary sensor unit containing the inductive sensor arrangement,

Thus it can be seen that there are a wide variety of different possible relative spatial arrangements of the controller, inductive sensing circuit, and portable handheld device in different examples.

According to advantageous embodiments, the apparatus may include means for processing signals sensed by the signal sensing means and deriving based on the sensed signals one or more physiological parameters.

Preferably, the portable handheld device is a mobile communication device, for example a mobile telephone device.

It may for example be a smartphone. The device could alternatively be a tablet computer for example.

Examples in accordance with a further aspect of the invention provide a method for controlling functionality of an inductive sensing circuit, and a portable handheld device having electromagnetic emitting functions,

the method comprising implementing a switching function for switching between two modes:

a first mode in which the controller communicates with the portable handheld device to cause deactivation of at least a portion of electromagnetic emissions of the device, and to concurrently communicate with the inductive sensing circuit to cause activation of the inductive sensing circuit;

a second mode in which the controller communicates with the portable device to cause activation of said at least portion of electromagnetic emissions of the device, and to concurrently cause deactivation of the inductive sensing circuit.

As discussed above, the switching between the modes may be performed responsive to a control command. The switching may be responsive to opening of a software application (app) on the portable handheld device for example. For example this might trigger generation of a control command for triggering a switching of the modes.

Examples in accordance with a further aspect of the invention also provide a computer program product comprising code means configured, when run on a processor, to cause the processor to perform a method in accordance with any example or embodiment outlined above or described below, or in accordance with any claim of this application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically illustrates basic principles of inductive sensing;
Fig. 2 shows components of an example apparatus in accordance with one or more embodiments of the present invention;
Fig. 3 schematically illustrates the two operation modes implemented by the switching function in embodiments of the present invention;
Fig. 4 outlines components of an example inductive sensing circuit in accordance with one or more embodiments of the present invention;
Fig. 5 schematically illustrates an example sensing assembly and apparatus according to one or more embodiments;
Fig. 6 schematically illustrates an example apparatus in accordance with one or more embodiments;
Fig. 7 schematically illustrates a further apparatus in accordance with one or more embodiments;
Fig. 8 schematically illustrates a further apparatus in accordance with one or more embodiments;
Fig. 9 schematically illustrates use of an example apparatus in accordance with one or more embodiments;
Fig. 10 schematically illustrates software and hardware architecture of an example apparatus in accordance with one or more embodiments;
Fig. 11 schematically illustrates software and hardware architecture of a further example apparatus in accordance with one or more embodiments;
Fig. 12 schematically illustrates software and hardware architecture of a further example apparatus in accordance with one or more embodiments;
Fig. 13 schematically illustrates components of an example inductive sensing circuit in accordance with one or more embodiments; and
Fig. 14 illustrates maximum allowable loop current as a function of operating frequency, for two antenna of different diameter, for the purpose of limiting the operating current of the antenna.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a switching mechanism for enabling use of an inductive sensing circuit in association with a portable handheld device having electromagnetic transmission functionality in a manner that avoids harmful interference of the electromagnetic transmissions of the portable device with the subject when medical sensing is being performed. In particular, embodiments provide a controller arranged to control switching between two modes: a first mode in which at least a portion of the transmission functionality of the portable device is deactivated and concurrently the inductive sensing circuit is activated; and a second mode in which the electromagnetic transmission functionality of the portable device is fully activated, and the inductive sensing circuit is deactivated. Thus embodiments provide a means of toggling between two modes, the modes configured to avoid simultaneous inductive sensing and full-power electromagnetic transmission of the portable handheld device.

In the first mode, just a portion of the electric electromagnetic transmission functionality of the portable device may be deactivated, for example just radio transmitters which are incompatible with electromagnetic compliance regulations may be deactivated.

As discussed above, a problem with using portable handheld unit such as a mobile phone for medical tests is that its standard electromagnetic (EM) emissions mean it is usually not compliant with EMC (electromagnetic compliance) regulations. These limit the allowable EM emissions of a medical device. Thus, use of a mobile phone for medical sensing applications is not possible without some adaptation of its normal transmission functionality. The controller according to embodiments of the present invention allows suppression of some transmission functionality automatically when inductive sensing using an inductive sensing circuit is to be performed.

For background, current significant electromagnetic compliance (EMC) regulations will now be briefly discussed. This is simply for background and context, and the ranges discussed are not intended to limit the invention. Clearly, the specific features of regulations can vary with time, and so the invention is not intended to be bound by specific numbers discussed below, or set out in any current regulations.

At the time of writing, a generally accepted standard (by both European and American regulatory authorities) for the emission of electromagnetic radiation by medical devices is the IEC 60601-1-2. It describes both immunity to, and emission of, electromagnetic radiation with the purpose of preventing electromagnetic disturbances of electrical or electronic equipment in clinical environments. Annex C of IEC 60601-1-2 provides guidance for classification according to CISPR 11.

For medical devices of class B (domestic environments such as the home) limits for electromagnetic emissions are set in terms of an allowable level of electromagnetic energy measured at a distance of 10 meters from the device. Table 1 below sets out a summary of the emission limits for different frequency ranges.

*Table 1: CISPR11 EMI limits for average emitted electric field strength at 10m distance for class B devices.*

| Frequency range | Limit for average emission at 10 meters dB ($\mu$V/m) |
| --- | --- |
| 81.848-134.786 | 25 |
| 134.786-136.414 | 45 |
| 136.414-230 | 25 |
| 230-1000 | 32 |

Radar systems have previously been investigated in academic settings with a view to measuring pulse rate and breathing rate. It is also been proposed to build radar systems into future domestic appliances for gesture detection (e.g. Project Soli), to enable hands-free operation. However, such radar systems do not comply with current compliance regulations for electromagnetic emissions, such as those outlined in Table 1 above.

Radar systems in general are not EMC compliant. They are therefore not suitable to be developed or used as medical devices. Although exemptions to EMC regulations have been granted on previous occasions, for example for MRI scanners or x-ray scanners, this has only been in cases where no alternative means exist for implementing the medical sensing functionality. However, for detecting pulse rate and breathing rate, this is not the case is there already exist other modalities for measuring these parameters. Thus exemptions are not likely to be forthcoming.

Further regulations currently exist in relation to the maximum allowable exposure of the human body to electromagnetic fields (EMF).

Based on scientific evidence, the International Commission on Non-Ionizing Radiation Protection (ICNIRP) provides guidelines for the maximum exposure of the human body to electromagnetic fields. Most regulatory authorities worldwide adopt these guidelines in their legislation, such as for example the European Council Recommendation 1999/519/EC.

Basic restrictions are provided for various quantities and various frequency ranges. Table 2 below sets out a summary.

*Table 2: Basic restrictions for electric, magnetic and electromagnetic fields from the European Council recommendations.*

| Frequency range | Magnetic flux density (ml) | Current density (mA/m$^2$) (rms) | Whole body average SAR (W/kg) | Localised SAR (head and trunk) (W/kg) | Localised SAR (limbs) (W/kg) | Power density, S (W/m$^2$) |
|---|---|---|---|---|---|---|
| 0 Hz | 40 | - | - | - | - | - |
| >0-1 Hz | - | 8 | - | - | - | - |
| 1-4 Hz | - | 8/f | - | - | - | - |
| 4-1 000 Hz | - | 2 | - | - | - | - |
| 1 000 Hz-100 kHz | - | f/500 | - | - | - | - |
| 100 kHz-10 MHz | - | f/500 | 0,08 | 2 | 4 | - |
| 10 MHz-10 GHz | - | - | 0,08 | 2 | 4 | - |
| 10-300 GHz | - | - | - | - | - | 10 |

Further technical and regulatory restrictions exist with respect to the allowable exposure of the human body to electromagnetic emissions in the case that an implantable medical device has been implanted in the body.

The European Active Implantable Medical Devices (AIMD) Directive (90/385/EEC) and associated standards (EN 45502-2-1: 2003; EN 45502-2-2:2008; EN 50527-1:2016) ensure that AIMDs operate uninfluenced as long as the General Public reference levels of the European Council recommendation (1999/519/EC) are not exceeded (except for static fields, and without any time averaging (EN 50527-1:2020 5.1.2)). The reference levels of 1999/519/EC are set out below in Table 3. These restrictions are tighter than the basic restrictions set out above in Table 2.

*Table 3: Reference levels for general public exposure to time-varying electric and magnetic fields (unperturbed root mean squared (rms) values). Frequency, f is as indicated in the corresponding row of the frequency range column. Source: Directive 1999/519/EC.*

| Frequency range | E-field strength (V m$^{-1}$) | H-field strength (A m$^{-1}$) | B-field strength (microTesla) |
|---|---|---|---|
| 1 - 8 Hz | 10,000 | $3.2 \times 10^4/f^2$ | $4 \times 10^4/f^2$ |
| 8-25 Hz | 10,000 | 4,000/f | 5000/f |
| 0.025-0.8 kHz | 250/f | 4/f | 5/f |
| 0.8 - 3 kHz | 250/f | 5 | 6.25 |
| 3 -150 kHz | 87 | 5 | 6.25 |
| 0.15 - 1 MHz | 87 | 0.73/f | 0.92/f |
| 1 - 10 MHz | 87/f½ | 0.73/f | 0.92/f |
| 10 - 400 MHz | 28 | 0.073 | 0.092 |

(continued)

| Frequency range | E-field strength (V m⁻¹) | H-field strength (A m⁻¹) | B-field strength (microTesla) |
|---|---|---|---|
| 400 - 2,000 GHz | $1.375f^{1/2}$ | $0.003f^{1/2}$ | $0.0046f^{1/2}$ |
| 2 - 300 GHz | 61 | 0.16 | 0.20 |

By way of illustration, for a sensor used as a stethoscope close to the thorax of a patient, the above reference levels significantly limit the maximum electromagnetic field that is permitted to be imposed on the patient. Various radio-systems present in current smartphones are non-compliant with these levels. Also, current known radar-based systems that might be applied to measure pulse and breathing would very likely exceed these reference levels when used close to the thorax of a patient.

As discussed above, embodiments of the present invention are based on control of operation of an inductive sensing circuit. Some embodiments include an inductive sensing circuit. Inductive sensing circuit preferably includes a resonator circuit having an antenna and signal generation means coupled to the antenna for driving the antenna to generate alternating electromagnetic fields or signals.

For purposes of further understanding the invention, the basic principles of inductive sensing, as referred to in the present disclosure, will now be briefly outlined.

Inductive sensing operates on the principle of inductive coupling, whereby a coil or wire has induced across it a potential difference due to exposure to a time varying magnetic field. Embodiments of the present invention use this principle to measure strength of electromagnetic signals generated within regions of a body by sensing changes in the inductance of a coil or loop antenna placed in proximity to the body, where these changes are detected based on changing resonance characteristics of the antenna or resonator circuit.

Certain embodiments of the present invention make use of a resonator circuit comprising an antenna (which may in preferred embodiments comprise only a single turn loop) to stimulate or excite a body with electromagnetic signals (waves) and to sense signals emitted back from the body in response to those excitation signals.

The coil may be driven with an alternating current to generate the excitation signals for application to the body. These may be propagating electromagnetic signals, propagated in to the medium in some cases, or the signals can consist of a non-propagating electromagnetic field applied to the medium, i.e. by bringing the loop antenna source into proximity of the target medium. The alternating current creates a field of alternating field strength.

When the coil is brought into proximity with a body, the inductance L acquires an additional reflected inductance component, $L_r$, arising due to eddy currents induced in the stimulated body as a result of application of the excitation signals.

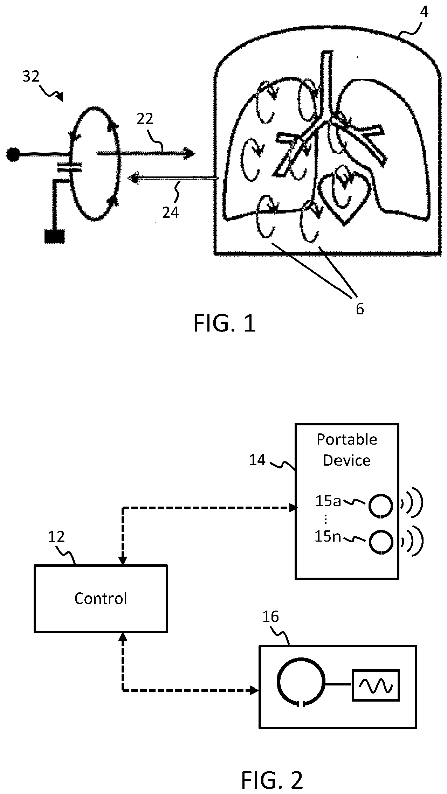

This is illustrated schematically in Fig. 1, which shows by way of example a loop antenna 32 being driven with an alternating current in proximity to a thorax 4 of a subject, so as to apply electromagnetic signals 22 to the thorax.

As a consequence, eddy currents 6 are induced within in the thorax.

These eddy currents in turn effectively make a contribution to the inductance of the loop antenna 32. This is because they themselves result in generation of a time-varying magnetic flux 24 of equivalent frequency to that generated by the primary antenna 32. These eddy-current fluxes combine with the primary flux of the antenna, resulting in a modified induced back-EMF in the antenna, and hence a larger measurable effective inductance.

The added component of inductance arising from the eddy currents is referred to as 'reflected inductance', $L_r$. The total inductance $Lr$ of the coil antenna 32 may be expressed as:

$$L_t = L_0 + L_r$$

where $L_0$ is the self-inductance of the coil antenna 32 in free space and $L_r$ is the reflected inductance caused by the presence of the proximate body.

In general, the reflected inductance, $L_r$, is complex, and can be expressed as

$$L_r = L_r' + iL_r''$$

where $L_r'$ is related to a reactive impedance of the coil antenna and $L_r''$ is related to resistive impedance of the coil.

The addition of the reflected component of inductance $L_r$ leads to a detuning of electrical characteristics of the resonator circuit. In particular, both the natural radial frequency of the resonator circuit and the damping factor of the resonator circuit change. By measuring this detuning of the electrical characteristics, the real and imaginary parts of the reflected inductance $L_r$ can be detected.

In particular, the real part of the additional inductance component, $L_r$, manifests in the frequency of the resonator circuit or antenna. The imaginary part of the additional inductance component manifests in the amplitude of the resonator circuit. Hence, by measuring changes in the frequency and amplitude of the resonator circuit (current), and deriving a first and second input signal respectively, signals indicative of the underlying anatomical movements and phenomena are detected.

An example arrangement in accordance with embodiments of the present invention is illustrated schematically in Fig. 2.

According to one aspect of the invention, there is provided a controller. An example controller 12 is shown in Fig. 2. The controller may comprise one or more control components or processors for example.

The controller comprises an input/output for receiving and transmitting data when the controller is communicatively coupled with an inductive sensing circuit and a portable handheld device, the portable handheld device having electromagnetic emitting functions. The portable handheld device may be a mobile communication device for example. The portable handheld device 14 may comprise a set 15a-15n of electromagnetic (EM) emitters or transmitters (such as antennas). Each of these may be controllable between an active (transmitting) state and a non-active (non-transmitting) state. They may emit at different frequencies or powers in certain examples.

The controller 12 is configured with a switching function wherein it controls switching between two modes.

In a first mode, the controller 12 is configured to communicate with the portable handheld device 14 to cause deactivation of at least a portion of electromagnetic emissions of the portable device, and to concurrently communicate with the inductive sensing circuit 16 to cause activation of the inductive sensing. For example, the controller may communicate with the portable handheld device 14 to cause it to de-activate a set of one or more of the electromagnetic transmitters 15a-15n, or to cause it to stop provision of one or more transmission signals provided to the transmitters by a controller of the portable handheld device.

The controller is configured to communicate with the inductive sensing circuit 16 to cause it to activate a signal generator in the circuit to trigger supply of a drive signal to an antenna in the inductive sensing circuit. Alternatively, the controller is configured to communicate with the portable device 14 to cause it to activate a power supply (not shown) from the portable device 14 to the inductive sensing circuit 60.

In a second mode, the controller 12 communicates with the portable device 14 to cause activation of said at least portion of electromagnetic emissions of the device, and to concurrently cause deactivation of the inductive sensing circuit 16.

The controller 12 may be provided by itself, and arranged in use for coupling with an inductive sensing circuit 16 and a portable handheld device 14 (via an input/output for receiving and transmitting data when coupled with the inductive sensing circuit and portable handheld device) and arranged for implementing the switching between the two modes. Alternatively, an apparatus may be provided which includes the controller 12 and one or both of the inductive sensing circuit and the portable handheld device. The controller may be integrated in one or other of the portable device and the inductive sensing circuit, and/or all three components may be integrated in a single device. These various options will be outlined in more detail later.

Thus, embodiments of the present disclosure provide control functionality configured such that for example whenever the inductive medical sensor 16 is activated for the purpose of measurement of pulse and breathing, a certain portion of the on-board radio transmitters (for example those that are incompatible with EMC regulations for medical devices) are automatically disabled at the same time.

The control functionality may be implemented with a software control switch or with a hardware switch, the switch in either case configured to regulate two things at the same time: (1) enabling/disabling the inductive medical sensor, and (2) disabling/enabling certain radio transmission functionality, e.g. a subset of transmitters that do not comply with EMC regulations for medical devices.

By combining these two functions in a single variable or switch, the risk that both the sensor and the harmful radio transmitters are enabled at the same time is mitigated.

The portable handheld device may in examples be a smartphone. Currently, smartphones can be set to airplane mode. Airplane mode is a separate switch that can be toggled by the user, but this switch is not coupled with enabling a sensor.

More generally, the portable handheld device may be a mobile communications device such as a smartphone. It may include a display and a user interface. It may include a touchscreen display for example. It may comprise local controllers or processors for facilitating local or native operations of the device. It includes a set of one or more electromagnetic emitters or transmitters 15a-15n which facilitate mobile communication functionality. These may be microwave emitters for facilitating cellular network communication, for example GPRS transmitters. They may include transmitters for enabling local area communication, e.g. Bluetooth communication. They may include radiofrequency transmitters, e.g. RFID transmitters, for enabling RFID functionality. They may include transmitters for enabling Wi-Fi communication.

In certain embodiments of the present invention, a global software setting may be added that enables the special medical sensor while - at the same time - disabling harmful radio-transmitters, while leaving the functionality of the airplane-mode setting unchanged. Thus the two modes in this case are implemented with a fully independent switch, independent of the airplane mode setting.

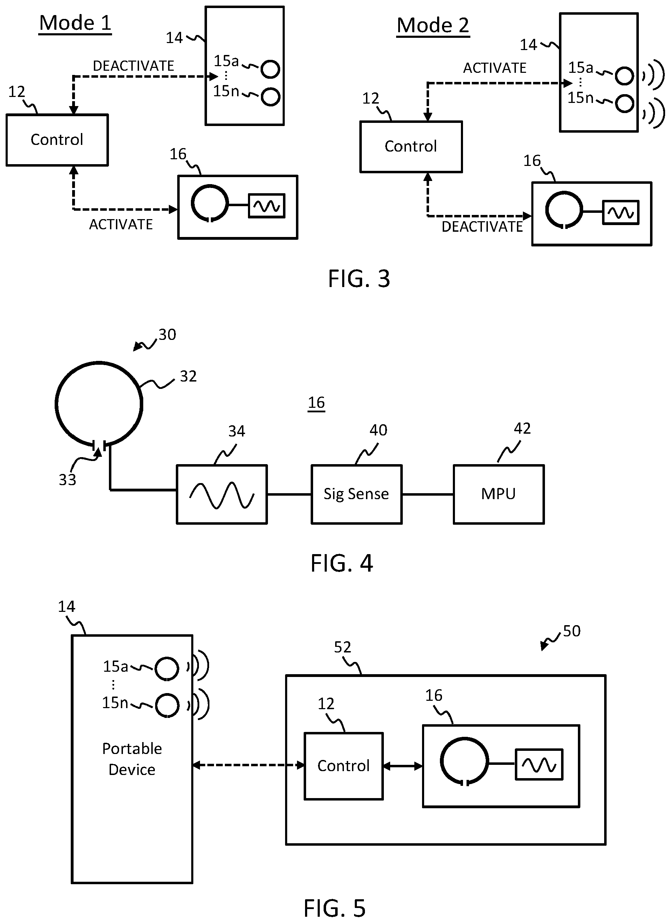

Fig. 3 illustrates the two switchable modes.

As illustrated, in Mode 1, the electromagnetic transmission functionality of the portable handheld device 14 is deactivated, while at the same time the inductive sensing circuit 16 is activated. Conversely, in Mode 2, the electromagnetic transmission functionality of the mobile portable handheld device 14 is activated, while at the same time the electromagnetic emissions of the inductive sensing circuit 16 are deactivated.

In certain embodiments, an apparatus may be provided which includes both the controller 12 and the inductive sensing circuit 16.

An example inductive sensing circuit 16 is schematically illustrated in Fig. 4.

The inductive sensing circuit 16 comprises a resonator circuit 30 comprising a loop antenna 32 and preferably an electrically coupled capacitor 33. The capacitance of the capacitor 33 may at least partially define a natural resonance frequency of the resonator circuit (in the absence of forcing or damping). When the antenna 32 is excited, it will tend to naturally resonate at the defined resonance frequency, generating electromagnetic signals at the same frequency. Selecting the capacitance of the capacitor hence allows for at least partial tuning of the frequency of the generated electromagnetic signals.

The inductive sensing circuit 16 preferably further includes a signal generation means 34 adapted to excite the loop antenna 32 to generate the electromagnetic excitation signals. The signal generation means may comprise a driver means for driving the antenna, for instance at a radial frequency $\omega$, i.e driving the antenna with an alternating current of frequency $\omega$. The driving means may be or comprise an oscillator for instance.

The signal generation means 34 may drive the antenna 32 of the resonator circuit 30 with a current of radial frequency $\omega$ where excitation signals of radial frequency $\omega$ are required.

By exciting the resonator circuit 30, a resonating current is induced to flow back and forth through the loop antenna into the capacitor. By driving an alternating current through the antenna generation of oscillatory electromagnetic signals (waves) may thereby be stimulated.

The same antenna 32 is preferably used to generate the excitation signals as is used to sense the electromagnetic signals received from the body in response.

For the avoidance of doubt 'electromagnetic excitation signals' simply means electromagnetic signals for applying to the body for the purpose of exciting or stimulating generation of eddy currents within the body for in turn stimulating emission of electromagnetic signals back out of the body which can be sensed by the sensing system.

By 'electromagnetic signals' may generally be meant electromagnetic radiation emissions or electromagnetic near-field oscillations or electromagnetic oscillations and/or electromagnetic waves.

The inductive sensing circuit 16 may in advantageous examples further comprise a signal sensing or signal pick-up means ("Signal Sense") 40 adapted to sense secondary electromagnetic signals returned from the body by eddy currents induced in the body by the primary excitation signals. Detection of the signals may be based on detecting variations in electrical characteristics of the resonator circuit 30. The signal sensing means may include signal processing or analysis means for detecting or monitoring electrical characteristics of the current in the resonator circuit 30.

For example, the signal sensing means 40 may be adapted to monitor at least a frequency of the resonator circuit current, and an amplitude of the resonator circuit current. These properties of the current will change in dependence upon the strength of the reflected electromagnetic signals returned from the body and detected at the antenna.

Sensing of these signal characteristics is preferably performed at the same time (i.e. simultaneously with) excitation of the antenna for generating the excitation signals. Hence signal transmission and sensing is preferably performed simultaneously.

The alternating electromagnetic fields generated by the inductive sensing circuit 16 may be configured so as to be sufficiently weak as to stay within the EMC regulations for medical devices. However due to the operating principle of inductive sensing, even these relatively weak signals are sufficient to detect pulsation of objects within the body, such as the heart or the inflation level of the lung, thereby permitting in use detection of vital signs such as a heart rate or breathing rate.

The inductive sensing circuit 16 may in certain examples further comprise a controller or microprocessor module 42 ("MPU"). The microprocessor module may be configured for controlling the operation of the inductive sensing circuit, for example controlling the signal generator 34 and/or the signal sensing means. It may control activation and/or deactivation of the signal generator. It may control a frequency of a drive signal generated by the signal generator 34. In certain examples, it may be configured with signal processing functionality, and be configured to process inductive signals detected or extracted by the signal sensing module 40, for deriving from them one or more physiological parameters. It may derive one or more physiological signals representative of one or more physiological phenomena, such as heart movement or lung movement. It may derive values or signals representative of one or more physiological parameters, e.g. vital signs.

By way of non-limiting example, physiological or anatomical parameters which the microprocessor may be configured for deriving in different examples may include: pulse rate, breathing rate, pulse waveform, breathing waveform, detection of presence or absence of a pulse, or other cardiac or respiratory parameters (e.g. cardiac output, stroke volume, breathing capacity/volume). Any other parameters may also be derived in further examples.

Deriving the physiological parameters may be based on application of suitable algorithms which may be pre-installed or pre-stored on the microprocessor unit for example.

Various options are possible with regards to the relative physical configuration of the controller, inductive sensing circuit, and portable handheld device.

In some embodiments, there may be provided an apparatus comprising the controller and the inductive sensing circuit. In some embodiments, there may be provided an apparatus comprising a controller 12 and the portable handheld device. In further examples, an apparatus may be provided comprising a controller, inductive sensing circuit 16 and a portable handheld device 14.

According to one set of embodiments, there may be provided an inductive sensing assembly 50 comprising a carrier (e.g. a substrate) 52 on which are mounted a controller 12 and inductive sensing circuit 16. The substrate may be or comprise a circuit board, for example a PCB.

An example inductive sensing assembly 50 is shown schematically in Fig. 5.

The assembly 50 may be in the form for example of a chip. The chip may be suitable for integration in a portable handheld device for example, or in a secondary device to which the portable handheld device is operatively coupleable in use. Interaction between the sensing assembly 50 and the portable handheld device 14 in use is schematically illustrated in Fig. 5. In Fig. 5, the inductive sensing assembly is illustrated as external of the portable handheld device 14.

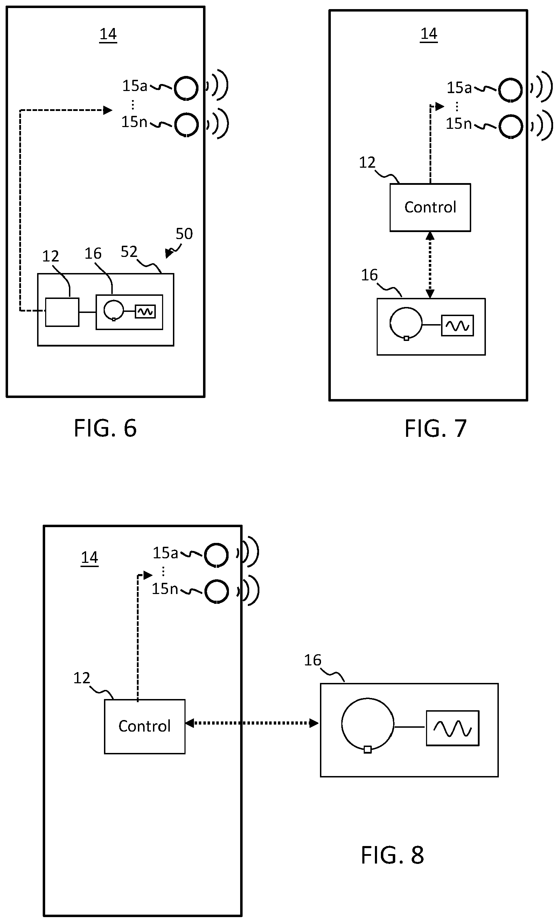

Fig. 6 schematically illustrates an example apparatus in accordance with one or more embodiments comprising a portable handheld device 14 in which an inductive sensing assembly 50 as illustrated in Fig. 5 is integrated in a portable handheld device 14. For example, the assembly 50 may be a chip formed of a substrate 52 to which the inductive sensing circuit 16 and controller 12 is mounted, and the chip installed integrally in the portable handheld device 14. The chip may for example be arranged to communicate with a set of electromagnetic transmitters 15 of the portable device to control activation and/or deactivation of at least a subset of the transmitters in the second and first modes respectively.

Other arrangements are also possible, Fig 7 illustrates a further example apparatus in accordance with one or more embodiments comprising a portable handheld device 14, a controller 12 integrated in the portable device 14 and an inductive sensing assembly also integrated in the device. In this example, the controller 12 and inductive sensing assembly are integrated in the portable device as separate components, i.e. not as part of a single integral circuit assembly or chip. For example, the controller may be provided by one or more processors or controllers integrated in the portable handheld device. These may be a dedicated one or more controllers or processors, or native or local controls or processors of the portable handheld device may be utilized to provide the control functionality of the controller 12.

A further possible arrangement is schematically illustrated in Fig. 8. The apparatus in this case comprises a portable handheld device 14 in which the controller 12 is integrated, but wherein the inductive sensing circuit 16 is provided separate to the portable handheld device. The inductive sensing circuit 16 may for example be integrated in a secondary unit, for example a peripheral or auxiliary sensing unit. This may in advantageous examples be provided physically coupleable in use with the portable handheld device. For example, in some cases it could be integrated in a sleeve article, the sleeve article shaped and configured to being wrapped removably around the portable mobile device 14 in use. In this way the inductive sensing circuit is provided associated with the portable handheld device, such that the two can be utilized together in concert. The secondary unit may take however any physical form, and is preferably provided with coupling means for removably or releasably mechanically coupling the secondary unit with the portable handheld unit.

According to any of the above-described arrangements, in use the controller 12 may be configured to control switching between the two modes responsive to receipt of one or more predefined control commands. For example these might be received from a controller or processor of the portable handheld device 14, for example triggered by manual activation by a user. This might be via pressing a button within a relevant app. Alternatively it might be triggered automatically by software of the portable handheld device 14.

In use, first, the controller 12 may switch the apparatus to the first mode, thereby deactivating a portion of the transmission functionality of the portable handheld device.

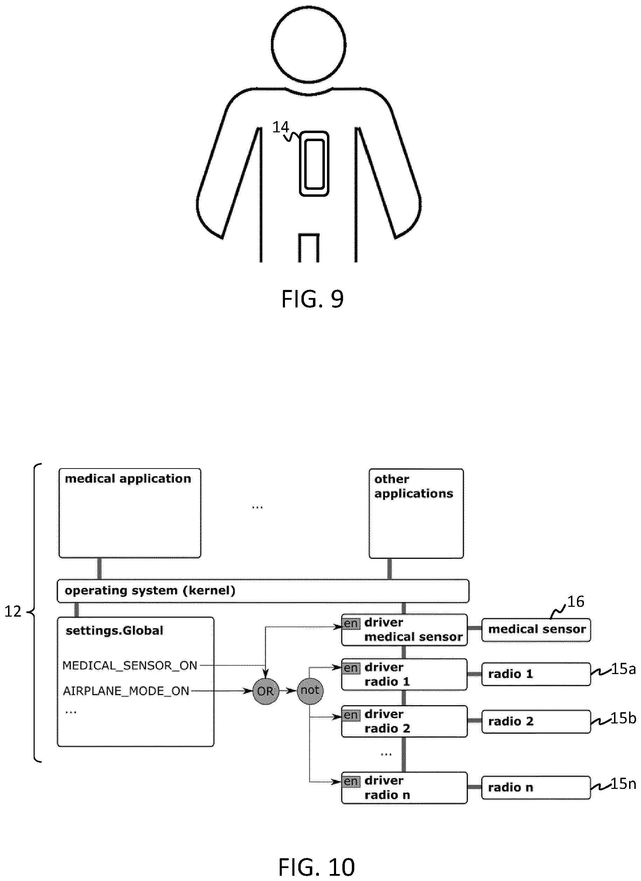

According to any of the above described arrangements, the user may then hold the apparatus including portable handheld device 14, inductive sensing circuit 16, and controller 12, contactlessly above a relevant region of the patient's body, such as the chest. This is illustrated in Fig. 9 for example. In this example, the apparatus is shown as a portable handheld device which has the controller 12 and inductive sensing circuit 16 integrated therein. The user then activates commencement of inductive sensing. For example, the portable handheld device controls operation of the inductive sensing circuit to acquire sensing signals. The sensing signals are processed to derive from them one or more physiological signals, for example pulse rate and breathing rate. The inductive sensing circuit may include a signal processing means for processing the inductive sensing signals to derive the one or more physiological parameters. Alternatively, inductive sensing signals may be communicated from the inductive sensing circuit to a controller or processor of the portable handheld device and processed by the portable handheld device to derive the physiological signals.

The switching function of the controller 12 may be implemented in numerous different ways. In preferred embodiments, it is implemented with software of the portable handheld device. In particular, it is preferably implemented using a software variable of software of the portable handheld device. This may be a global software variable or a software variable which is local to a particular application associated with the switching function or with the inductive sensing.

For example, in Android systems, the global settings are located in settings. Global. For purposes of illustration, the software variable associated with the two modes may be named "MEDICAL_SENSOR". When the apparatus is in Mode 1, this variable is set to "MEDICAL_SENSOR_ON". When the apparatus is in Mode 2, this variable is set to "MEDICAL_SENSOR_OFF". The controller 12 may be configured to implement switching between modes 1 and 2 responsive to, or dependent on the value of, this software variable.

The advantage of using a global variable or setting is that other applications cannot change the mode unilaterally. Hence, this is the most secure approach.

This approach also means that the application installed on the portable handheld device for controlling operation of the medical sensing requires a system privilege to set the global setting. Normal applications (i.e. downloaded from a public application directory such as Google Play) have no system rights to set global settings of the Android operating system. The manufacturer of the portable handheld device however can ship a preconfigured application that does have those privileges pre-assigned (possible from Android 5.0 and later). Thus for example, according to one or more embodiments, an apparatus may be provided having a portable handheld device on which there is preinstalled a medical application having system rights to set the MEDICAL_SENSOR global setting. This leads to a very secure approach.

Fig. 10 schematically illustrates the architecture of an example apparatus in accordance with one or more embodiments. The apparatus in this case is assumed to comprise a portable handheld device 14 having an integrated controller 12 and inductive sensing circuit 16 ("medical sensor"). The portable handheld device 14 also comprises a set of internal electromagnetic transmitters ("radio") 15 which are controllable by the controller 12.

The controller is in this case assumed to be a native or local controller or processor of the portable handheld device, for example a mother processor of the device. Thus, the operating software of the portable device implements the control functionality of the controller 12 in this example, i.e. controls switching between the two modes, Mode 1 and Mode 2. Thus the controller 12 in Fig. 10 is illustrated in terms of software components of the handheld portable device 14. These software elements include a dedicated application ("medical application") installed for controlling functionality of the inductive sensing circuit 16, as well as the operating system of the portable handheld device 14, as well as a set of global settings ("settings.global").

The apparatus of Fig. 10 is shown having a global variable or setting ("MEDICAL_SENSOR_ON") associated with the switching function. As shown, this is a separate global variable to the airplane mode ("AIRPLANE_MODE_ON") meaning that the latter operates independently of the inductive sensor modes. This means that the electromagnetic transmitters 15 cannot be activated without both AIR_PLANE_MODE_ON and MEDICAL_SENSOR_ON being set to false.

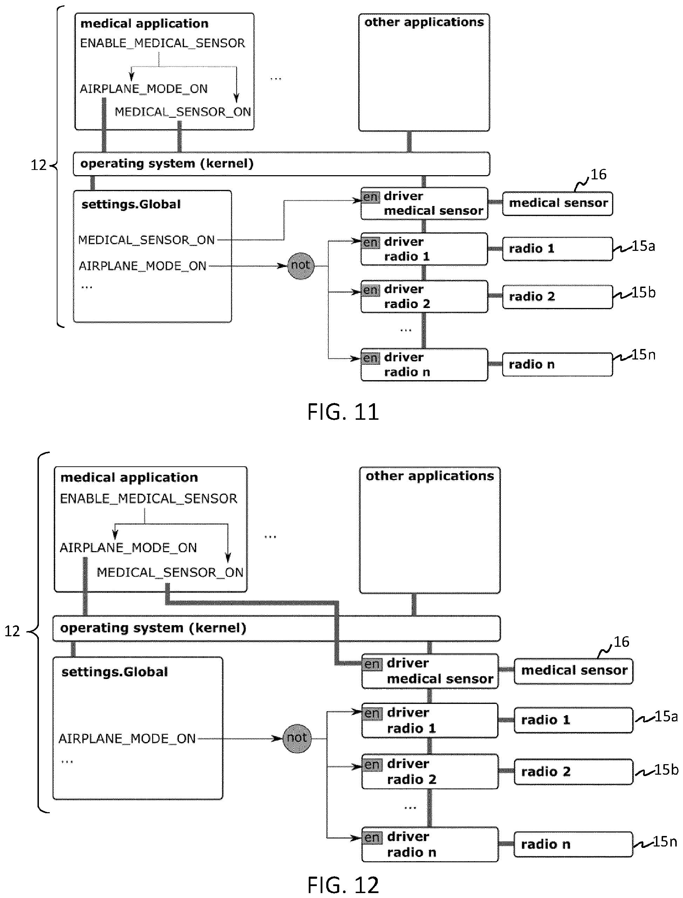

An alternative software based implementation of the switching function of the controller is illustrated schematically in Fig.11. In this example, the apparatus is assumed to be the same as that of Fig.10, comprising a portable handheld device 14, having an integrated inductive sensing circuit 16 and wherein a native or local controller or processor of the portable device functions as the controller 12 of the apparatus. Again, the software components of the portable device include a dedicated application for controlling the inductive sensing circuit ("medical application"), the operating system of the device, and a set of global settings.

In this example, the software variable associated with the different inductive sensing modes (Mode 1, Mode 2) is located in memory space of the dedicated application for the inductive sensing ("medical application").

An advantage of the embodiment of Fig 11 is that there is no need to add a settings. Global variable MEDICAL_SENSOR_ON to the collection of global settings. The embodiment still employs a global variable, in the form of AIRPLANE_MODE_ON, which is used to deactivate and reactivate the radio transmitters of the mobile device 14. Use of the global variable for this purpose ensures that normal applications (i.e., application without privileges to modify global settings) cannot override the action of the medical application in deactivating the radio transmitters of the portable handheld device 14.

A further alternative software based implementation of the switching function of the controller 12 is illustrated schematically in Fig. 12. In this example, the apparatus is again assumed to be the same as that of Fig. 10 and Fig. 11, comprising a portable handheld device 14, having an integrated inductive sensing circuit 16 and wherein a native or local controller or processor of the portable device functions as the controller 12 of the apparatus.

In this example, the single software variable ("MEDICAL SENSOR ON") is located in the memory space of a dedicated application for controlling the inductive sensing ("medical application"). In this embodiment, there is no global variable to enable or disable the medical driver. Instead, enabling or disabling the medical sensor can be done by normal applications. This is less secure than the implementations of Fig. 10 and Fig. 11, but still provides the primary advantages of the inventive concept in that a user is unable to accidentally activate the medical sensing functionality while the radio transmitters of the portable mobile device are active.

In accordance with one or more embodiments, there may be provided means for generating an automatic reminder for the purpose of alerting a user in case they forget to switch the apparatus back from the inductive sensing mode (mode 1) to the normal operation mode (mode 2). The automatic reminder may be implemented by the controller 12, or by software of the portable handheld device 14 in further examples. The automatic reminder may be configured for example to trigger after a certain period of user inactivity (e.g. user interface inactivity, or accelerometer inactivity), and/or after a certain period of absence of any detectable physiological signals in the inductive sensing signal of the inductive sensing circuit 16.

As discussed above, the inductive sensing circuit 16 preferably comprises a resonator circuit, the resonator circuit comprising an antenna 32. The resonator circuit may further include a capacitor in some examples, or the resonator circuit may include no capacitor and may be self-tuning. Preferably, the inductive sensing circuit further comprises a signal generator 34 configured to generate an alternating drive signal for driving the antenna to oscillate at a resonant frequency of the resonant circuit. The antenna 32 is preferably a loop antenna with a single loop or winding.

A frequency range at which the resonator circuit is driven may preferably be between 30-1000 MHz. Advantageous frequency ranges for the inductive sensor were discussed in detail in WO 2018/127482 for example.

In some examples, a multifrequency drive scheme may be implemented in which the resonator circuit is driven at multiple frequencies either simultaneously or sequentially. Different frequencies can permit penetration to different depths within the body, or can be useful for inducing a response in different types of tissue.

There is preferably included a current limiting means for limiting the maximum driving current of the resonator circuit, to thereby limit the maximum electromagnetic output power of the inductive sensing circuit. This thereby enables compliance with EMC regulations. Optionally the current limiting means may permit adjustment of the maximum driving current of the resonator circuit, for instance in case regulations change, or for compliance with regulations in different parts of the world.

Optionally the loop antenna 32 may be a multipurpose antenna configured for performing multiple functionalities. For instance it could be an integrated loop antenna of the mobile portable device 14 provided for wireless charging functionality. The same inductive loop may be utilized in accordance with embodiments of the present invention to perform the functions of the antenna of the inductive sensing circuits. This thereby saves space and reduces the total number of components in the mobile portable device 14, meaning overall form factor of the mobile portable device can be reduced or space is freed for more components for further functions.

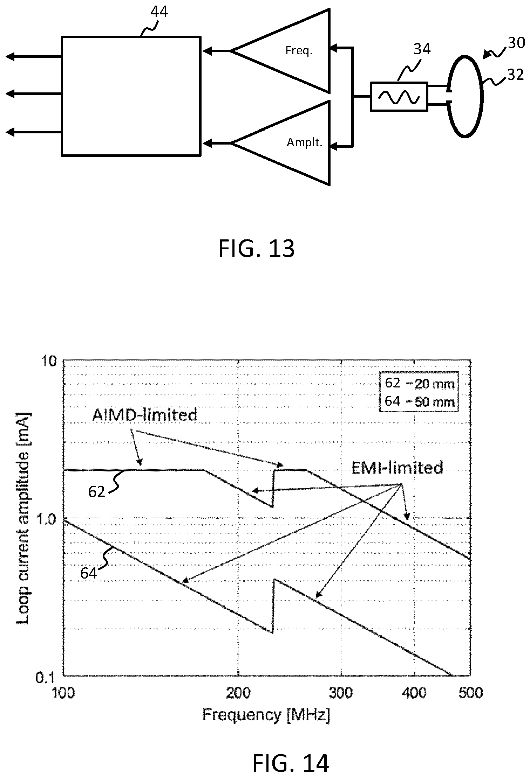

The architecture of an example inductive sensing circuit is schematically illustrated in fig 13. The circuit comprises a resonator circuit 30, comprising an antenna 32 with a capacitor. Coupled to the antenna is a signal generator 34, for example an oscillator. The signal generator is configured to drive the antenna with an alternating signal to cause resonance of the antenna to thereby generate electromagnetic excitation signals for application to the body. Signals returned from the body are simultaneously sensed by the antenna. The signal sensing means (not shown) may be provided for detecting the returned signals at the antenna.

The detection may be based on analyzing changes in one or more electrical characteristics of the resonator circuit 30, such as a resonant frequency of the resonator circuit or a natural amplitude of the resonator circuit. Separate signals corresponding to these electrical characteristics can be extracted. This is illustrated schematically in Fig. 13, wherein by way of illustration two signals are extracted corresponding to variations in a natural frequency of the resonator circuit and variations in a natural amplitude of the resonator circuit over time. These two signals correspond to a real and imaginary part of the impedance of the resonator circuit. Thus by measuring the frequency and amplitude variations, effectively variations in impedance of the loop are measured.

These two signals are output to a signal processing unit 44 which processes the signals to derive from them measures indicative of one or more physiological parameters. For example it may derive from one or both of the signals measures or signals indicative of a heartrate and/or a breathing rate of the subject. Although not shown in Fig. 13, the inductive sensing circuit 16 may further be provided with a controller for example a microprocessor unit 42, configured to control operation of the inductive sensing circuit. For example this microprocessor may control operation of the signal generator 34 and/or the signal processing unit 44. It may control a frequency at which the signal generator 34 generates drive signals for driving the resonator circuit, to thereby adjust an operating frequency of the resonator circuit. Alternatively, operation of the inductive sensing circuit 16 may be controlled by the controller 12 of the apparatus, or by a controller or processor of the portable handheld device 14.

An optimal frequency for drive signals for driving the antenna may depend upon the diameter of the antenna used.

It has been found that for sensing heart rate and/or breathing rate, it is optimal to use a loop antenna having a diameter of approximately 4-6 cm. The corresponding optimal frequency range for this diameter range can be computed by using a normalized radial frequency of $\hat{\omega}$ = 0.04 - 0.25, which corresponds to a frequency range of between approximately 67 MHz and 625 MHz. Normalized frequency, $\hat{\omega}$, is defined as $\widehat{\omega} \equiv \dfrac{\omega}{\omega_{ref}}$ , where $w_{ref} = 2\pi c/l$, where $l =$ circumferential length of the single loop of the antenna, and c = the speed of light.

Particularly effective functionality has been found to be provided by operating frequencies in the range of approximately 150 MHz to 300 MHz, although frequencies outside this range are also effective.

As mentioned above, the operating frequency of the resonator circuit can be switched between different values in a sequential manner in some examples. The different operating frequencies can be switched between in a relatively fast manner for example to implement a sweep of frequency values. In this example, effectively signals from multiple frequencies can be captured in a time multiplexed manner. This has the advantage of increasing a dimensionality of the signal, which can be utilized to separate signal components originating from different physiological sources (e.g. breathing, pulse, body motion, other noise sources.).

As mentioned above, the inductive sensing circuit can include means for limiting the maximum operating current of the resonator circuit, to limit the maximum electromagnetic output power of the inductive sensing circuit. This enables compliance with EMC regulations.

The maximum allowable loop current for a given set maximum output power depends upon the diameter of the loop antenna 32 and upon the intended operating frequency for the resonator circuit.

The maximum loop current as a function of frequency for antenna 32 of two different diameters is depicted in the graph of Fig. 14. Line 62 corresponds to a loop antenna 32 of diameter 20 mm. Line 64 corresponds to a loop antenna of diameter 50 mm. Indicated on the graph are the regions of each of the lines which are compliant with AIMD regulations (see Table 3 above) and EMI regulations (see Table 1 and Table 2 above) respectively.

Limiting the current in the resonator circuit without impacting unduly on the sensing performance of the circuit is not straightforward. This is because current limiters typically induce additional noise and reduce the sensitivity of the inductive sensing circuit.

A number of different approaches are possible for limiting the current of the resonator circuit 30.

According to a first approach, the bias current of the signal generator 34 (oscillator) may be limited. Limiting the bias current has the effect of reducing the voltage swing on the resonating LC circuit, and therefore reduces the open-loop gain of the oscillator electronics. This in turn will result in a more limited loop current.

The bias current can be (actively) limited through a number of means.

These include for example increasing the impedance at source/emitter or drain/collector, and/or decreasing the supply voltage provided to the oscillator 34.

In particular, a balanced oscillator circuit may contain at least two transistors. The signal generator (oscillator) bias current is the sum of the drain-source currents (in case of field-effect transistors) or the collector-emitter currents (in case of bipolar junction transistors). The bias current can be controlled by adding a resistor in these current paths. For example, fixed resistors could be added to the drains of the FETs or fixed resistors could be added to the collectors of the BJTs. More generally, these resistors could be controlled or variable resistors such as voltage-controlled resistors, thereby permitting dynamic adjustment of the bias current depending upon the actual oscillation amplitude.

Thus, the oscillator can be configured to have an adjustable bias voltage, instead of having one fixed value. Control of the bias voltage may be implemented in both the analog domain and the digital domain. Controlling the bias current is useful for maximizing signal quality while still meeting EMI requirements.

A second approach to limiting the resonator circuit 30 current is to increase the impedance of the loop antenna 12 and/or the resonator circuit 30. With the same supply voltage, a higher loop impedance will result in a lower loop current. Two means of increasing the loop impedance include: adding series inductance to the antenna loop, and/or adding series resistance to the loop antenna.

Adding series inductance may be preferable since an inductor adds less noise to the signal (assuming a high Q inductor). One downside of these solutions is that the sensitivity to changes in inductance and resistance of the resonator circuit, i.e. changes in frequency and amplitude, is reduced as well. In the event that these electrical characteristics are used for detecting the physiological signals, this means the inductive sensor sensitivity is reduced.

A further possible means of increasing the antenna 32 impedance is to add a transformer between the antenna 32 and the signal generator 34 electronics. The ratio between the antenna current and the current in the signal generator can now be tuned such that the antenna current falls below a defined maximum (i.e. so that it can meet defined EMI regulations), and so that the oscillator 34 has a stable operating point. For example, a low loop current and a high oscillator currents can be simultaneously achieved.

In particular, a stable operating point means that the current amplitude of the signal generating circuit is sufficiently high to generate a stable, i.e., low-noise, oscillating signal. Oscillators typically become unstable when the current amplitude becomes too small, i.e., in a regime where the oscillator can shut down or the amplitude of the oscillation becomes noisy. For low-noise purposes, it is beneficial to have a sufficiently high current in the oscillator, which is referred to as the ideal operating point. Since this current is typically in excess of the maximum loop current, it is desirable to add the transformer in between the signal generating oscillator and the loop. In this way, the operating point (by which is meant the oscillation current) of the signal generating oscillator can be sufficiently high, while the operating point (oscillation current) of the loop is sufficiently low to meet EMC requirements of the loop.

In different examples, the transformer may be for instance an air-core transformer, or a ferrite core transformer.

One disadvantage of a transformer compared to use of a series inductance or resistance is that a transformer cannot be controlled to actively alter the value of the loop impedance and therefore the sensor could be operated at a non-ideal operating point.

A further possible approach to limiting the antenna 32 current is to control the open loop gain of the oscillator. This can decrease the voltage swing on the resonating LC circuit, and therefore decrease the current through the antenna loop 32.

One possible approach to controlling the open loop gain is to alter the DC operating point of the transistors used in the oscillator 34. The operating point may for example be moved to a value falling at a less steep or steeper part of the transconductance curve for the transistor, thereby controlling the open loop gain of the oscillator. The DC operating point can alternatively be actively controlled. A disadvantage of active control is that noise sources in the circuit of the oscillator are less suppressed by the loop gain, therefore reducing the signal quality of the measurement.

Examples in accordance with a further aspect of the invention provide a method for controlling functionality of an inductive sensing circuit, and a portable handheld device having electromagnetic emitting functions, the method comprising implementing a switching function for switching between two modes:

a first mode in which the portable handheld device is controlled to cause deactivation of at least a portion of electromagnetic emissions of the device, and, concurrently, the inductive sensing circuit is controlled to cause activation of the inductive sensing circuit;

a second mode in which the portable device is controlled to cause activation of said at least portion of electromagnetic emissions of the device, and concurrently the inductive sensing circuit is controlled to cause deactivation of the inductive sensing circuit.

Implementation options and details for each of the above steps may be understood and interpreted in accordance with the explanations and descriptions provided above for the apparatus aspect of the present invention (i.e. the controller aspect).

Any of the examples, options or embodiment features or details described above in respect of the apparatus aspect of this invention (in respect of the controller) may be applied or combined or incorporated mutatis mutandis into the present method aspect of the invention.

Examples in accordance with a further aspect of the invention also provide a computer program product comprising code means configured, when run on a processor, to cause the processor to perform a method in accordance with any example or embodiment outlined above or described below, or in accordance with any claim of this application.

Embodiments of the invention provide numerous advantages compared to known medical sensing devices and systems. A summary of the main advantages will now be outlined.

One advantage is that embodiments enable medical sensing utilizing, or associated with, a portable handheld device such as a mobile phone without contravening EMC regulations (discussed above). In particular, the switching mechanism provides a means by which it can be ensured that harmful radio transmitters of the portable handheld device are disabled automatically whenever the inductive sensing circuit is enabled. This ensures improved safety of the apparatus and opens the possibility of regulatory approval of the apparatus to be used as a medical device.

This automatic disablement of the pre-defined portion of the mobile device EM emissions also enables avoidance of any electromagnetic interference between the EM emissions of the mobile device and the inductive sensor. Thus, the inductive sensing can be performed free from interference from the mobile device, avoiding deterioration or corruption of the inductive sensing signal.

Furthermore, the other functions of the portable medical device can still be used in full when in the second mode, i.e. non-sensing mode. For example, once a measurement has been performed using the inductive sensing circuit, the communication functionality of the portable handheld device can be used to communicate or transmit the recorded measurement via the on-board radio transmitters.

Embodiments of the invention, in use, can ensure that a patient is not exposed to electromagnetic fields that exceed safe limits for electromagnetic radiation. As discussed above, this is especially important for patients with an implantable medical device such as a pacemaker. Often an operator may not know at the time of performing a measurement whether a patient has an implantable device such as a pacemaker or not. Thus it is advantageous to have functionality which automatically ensures that potentially dangerous EM emissions are deactivated whenever an inductive sensing measurement is performed.

A further advantage is that a user is not burdened with a requirement to manually disable radio transmitters when using the inductive sensor (since the controller ensures this happens automatically when the inductive sensor is enabled in Mode 1).

Furthermore, inductive sensing is a modality that is relatively simple to operate (compared for example to other modalities such as ultrasound, MRI, or even traditional stethoscopes). In particular, non-trained or non-expert personnel can easily use an induction-based sensor for performing measurement. It can also be used in challenging circumstances, e.g. obese or overweight people, motion of the patient, poor lighting, or thick clothing.

A further advantage of inductive sensing as a modality is that it is contactless, avoiding risk of cross-contamination.

As discussed above, embodiments make use of controller. A controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

**Claims**

1. A controller (12),

   the controller comprising an input/output for receiving and transmitting data when the controller is communicatively coupled with an inductive sensing circuit (16) and a portable handheld device (14), the portable handheld device having electromagnetic emitting functions, and
   the controller operable to implement a switching function for switching between two modes:

   a first mode in which the controller is configured to communicate with the portable handheld device to cause deactivation of at least a portion of electromagnetic emissions of the device, and to concurrently communicate with the inductive sensing circuit to cause activation of the inductive sensing circuit; and
   a second mode in which the controller is configured to communicate with the portable handheld device to cause activation of said at least portion of electromagnetic emissions of the device, and to concurrently cause deactivation of the inductive sensing circuit,
   **characterized in that**, in the first mode, the controller is configured to cause the portable handheld device to activate a power supply from the portable handheld device to the inductive sensing circuit or alternatively to cause the inductive sensing circuit to activate a signal generator in the inductive sensing circuit to trigger supply of a drive signal to an antenna in the inductive sensing circuit, and wherein, in the second mode, the controller is configured to cause the portable handheld device to deactivate said power supply from the portable handheld device to the inductive sensing circuit.

2. The controller (12) as claimed in claim 1, wherein the first mode comprises causing deactivation of one or more electromagnetic transmitters of the portable device.

3. The controller (12) as claimed in claim 1 or 2, wherein the first mode comprises causing cessation of emission in one or more electromagnetic frequency bands or ranges, and/or causing cessation of any electromagnetic emissions above a defined threshold power.

4. The controller (12) as claimed in claim 1, further configured to receive an inductive sensing signal input from the inductive sensing circuit and to derive from the signal one or more physiological parameters, for example heart rate and/or breathing rate.

5. The controller (12) as claimed in claim 1, wherein the controller is configured to implement switching between the two modes responsive to receipt of one or more pre-defined control commands, and preferably wherein the controller is arranged to receive said control commands from the portable handheld device.

6. An inductive sensing assembly (50) comprising:

an inductive sensing circuit (16) for sensing electromagnetic signals returned from a body responsive to application of electromagnetic excitation signals to said body,
the inductive sensing circuit comprising a resonator circuit (30) comprising a loop antenna (32), the resonator circuit for generating said excitation signals when driven with a drive signal,
a controller (12) as claimed in any of claims 1-5, operatively coupled with the inductive sensing circuit; and
a carrier (52), the inductive sensing circuit and controller being mounted to the carrier.

7. The assembly (50) as claimed in claim 6, wherein the inductive sensing circuit comprises a signal generator for generating a drive signal for driving said antenna to generate said electromagnetic excitation signals, and optionally wherein the inductive sensing circuit comprises a signal sensing or pick-up means for detecting signals returned from the body based on detecting variations in one or more electrical characteristics of the resonator circuit.

8. An apparatus for inductive sensing, comprising:

a portable handheld device (14); and
a controller (12) as claimed in any of claims 1-5.

9. The apparatus as claimed in claim 8, further comprising an inductive sensing circuit (16) operatively coupled with the controller (12), the inductive sensing circuit for sensing electromagnetic signals returned from a body responsive to application of electromagnetic excitation signals to said body, and
the inductive sensing circuit comprising a resonator circuit (30) comprising a loop antenna (32), the resonator circuit for generating said excitation signals when driven with a drive signal.

10. The apparatus as claimed in claim 8 or 9, wherein

the inductive sensing circuit (16) is integrated in the portable handheld device (14), for example as part of a dedicated chip, or
the apparatus includes a secondary unit, the inductive sensing circuit being integrated in the secondary unit, and the portable handheld device being operatively coupled with the secondary unit.

11. The apparatus as claimed in any of claims 8-10,
wherein the apparatus comprises an inductive sensing assembly as claimed in any of claims 7-10, the controller and inductive sensing circuit (16) being provided by said inductive sensing assembly (50), and the inductive sensing assembly being operatively coupled with the portable handheld device (14).

12. The apparatus as claimed in any of claims 8-11, wherein

the apparatus includes a signal sensing or pick-up means (40) for detecting signals returned from the body based on detecting variations in one or more electrical characteristics of the resonator circuit (30), and
the apparatus includes means for processing signals sensed by the signal sensing means and deriving based on the sensed signals one or more physiological parameters.

13. The apparatus as claimed in any of claims 8-12, wherein the portable handheld device (14) is a mobile communication device, for example a mobile telephone device.

14. A method for controlling functionality of an inductive sensing circuit (16), and a portable handheld device (14) having electromagnetic emitting functions,
the method comprising implementing a switching function for switching between two modes:

a first mode in which the portable handheld device is controlled to induce deactivation of at least a portion of electromagnetic emissions of the device, and, concurrently, the inductive sensing circuit is controlled to induce activation of the inductive sensing circuit;

a second mode in which the portable device is controlled to cause activation of said at least portion of electromagnetic emissions of the device, and, concurrently, the inductive sensing circuit is controlled to induce deactivation of the inductive sensing circuit,

**characterized in that**, in the first mode, the portable handheld device is caused to activate a power supply from the portable handheld device to the inductive sensing circuit or alternatively to cause the inductive sensing circuit to activate a signal generator in the inductive sensing circuit to trigger supply of a drive signal to an antenna in the inductive sensing circuit, and wherein, in the second mode, the portable handheld device is caused to deactivate said power supply from the portable handheld device to the inductive sensing circuit.

15. A computer program product comprising code means configured, when run on the controller of any of the apparatus as claimed in claims 9-13, to cause the controller to perform the method of claim 14.

**Patentansprüche**

1. Steuerung (12);

   die Steuerung umfassend einen Eingang/Ausgang zum Empfangen und Senden von Daten, wenn die Steuerung kommunikativ mit einer induktiven Erfassungsschaltung (16) und einem tragbaren Handgerät (14) gekoppelt ist, wobei die tragbare handgehaltene Vorrichtung elektromagnetische Sendefunktionen aufweist, und die Steuerung betrieben werden kann, um eine Schaltfunktion zum Umschalten zwischen zwei Modi zu implementieren:

   einen ersten Modus, in dem die Steuerung konfiguriert ist, um mit der tragbaren handgehaltenen Vorrichtung zu kommunizieren, um eine Deaktivierung von mindestens einem Teil der elektromagnetischen Emissionen der Vorrichtung zu bewirken, und gleichzeitig mit der induktiven Sensorschaltung zu kommunizieren, um die Aktivierung der induktiven Sensorschaltung zu bewirken; und

   einen zweiten Modus, in dem die Steuerung konfiguriert ist, um mit der tragbaren handgehaltenen Vorrichtung zu kommunizieren, um eine Aktivierung des mindestens einen Teils der elektromagnetischen Emissionen der Vorrichtung und gleichzeitig die Deaktivierung des induktiven Sensorkreises zu bewirken, **dadurch gekennzeichnet, dass** die Steuerung in der ersten Betriebsart konfiguriert ist, um die tragbare handgehaltene Vorrichtung zu veranlassen, eine Stromversorgung von der tragbaren handgehaltenen Vorrichtung zu der induktiven Erfassungsschaltung zu aktivieren, oder alternativ die induktive Erfassungsschaltung zu veranlassen, einen Signalgenerator in der induktiven Erfassungsschaltung zu aktivieren, um die Zufuhr eines Treibersignals zu einer Antenne in der induktiven Erfassungsschaltung auszulösen, und wobei die Steuerung in der zweiten Betriebsart konfiguriert ist, um die tragbare handgehaltene Vorrichtung zu veranlassen, die Stromversorgung von der tragbaren handgehaltenen Vorrichtung zu der induktiven Erfassungsschaltung zu deaktivieren.

2. Steuerung (12) nach Anspruch 1, wobei der erste Modus ein Deaktivieren eines oder mehrerer elektromagnetischer Sender der tragbaren Vorrichtung umfasst.

3. Steuerung (12) nach Anspruch 1 oder 2, wobei der erste Modus eine Beendigung der Emission in einem oder mehreren elektromagnetischen Frequenzbändern oder -bereichen und/oder die Beendigung jeglicher elektromagnetischer Emission oberhalb einer definierten Schwellenwertleistung umfasst.

4. Steuerung (12) nach Anspruch 1 die ferner konfiguriert, um ein induktives Erfassungssignal von der induktiven Erfassungsschaltung zu empfangen und aus dem Signal einen oder mehrere physiologische Parameter, beispielsweise die Herzfrequenz und/oder die Atemfrequenz, abzuleiten.

5. Steuerung (12) nach Anspruch 1, wobei die Steuerung konfiguriert ist, um als Reaktion auf einen Empfang eines oder mehrerer vordefinierter Steuerbefehle zwischen den zwei Modi umzuschalten, und wobei die Steuerung vorzugsweise angeordnet ist, um die Steuerbefehle von der tragbaren handgehaltenen Vorrichtung zu empfangen.

6. Induktive Sensoranordnung (50), umfassend:

eine induktive Sensorschaltung (16) zum Erfassen von elektromagnetischen Signalen, die von einem Körper als Reaktion auf das Anlegen von elektromagnetischen Erregungssignalen an den Körper zurückgegeben werden,

die induktive Sensorschaltung umfassend eine Resonatorschaltung (30) umfassend eine Schleifenantenne (32), wobei die Resonatorschaltung zum Erzeugen der Erregungssignale dient, wenn sie mit einem Ansteuersignal angesteuert wird,

eine Steuerung (12) nach einem der Ansprüche 1 bis 5, die funktionsfähig mit der induktiven Sensorschaltung gekoppelt ist; und

einen Träger (52), wobei die induktive Sensorschaltung und die Steuerung auf dem Träger montiert sind.

7. Anordnung (50) nach Anspruch 6, wobei die induktive Sensorschaltung einen Signalgenerator zum Erzeugen eines Treibersignals zum Ansteuern der Antenne umfasst, um die elektromagnetischen Anregungssignale zu erzeugen, und wobei die induktive Sensorschaltung optional eine Signalerfassungs- oder -aufnahmeeinrichtung zum Erfassen von vom Körper zurückgegebenen Signalen basierend auf der Erfassung von Änderungen einer oder mehrerer elektrischer Eigenschaften der Resonatorschaltung umfasst.

8. Vorrichtung zum induktiven Abtasten, umfassend: eine tragbare handgehaltene Vorrichtung (14); und eine Steuerung (12) nach einem der Ansprüche 1-5.

9. Vorrichtung nach Anspruch 8, ferner umfassend eine induktive Sensorschaltung (16), die funktionsfähig mit der Steuerung (12) gekoppelt ist, wobei die induktive Sensorschaltung zum Erfassen elektromagnetischer Signale dient, die von einem Körper als Reaktion auf das Anlegen elektromagnetischer Anregungssignale an den Körper zurückgegeben werden, und

die induktive Sensorschaltung eine Resonatorschaltung (30) mit einer Schleifenantenne (32) umfasst, wobei die Resonatorschaltung zur Erzeugung der Erregungssignale dient, wenn sie mit einem Ansteuersignal angesteuert wird.

10. Vorrichtung nach Anspruch 8 oder 9, wobei die induktive Sensorschaltung (16) in die tragbare handgehaltene Vorrichtung (14) integriert ist, z.B. als Teil eines dedizierten Chips, oder

die Vorrichtung eine sekundäre Einheit umfasst, wobei die induktive Abtastschaltung in die sekundäre Einheit integriert ist und die tragbare handgehaltene Vorrichtung funktionsfähig mit der sekundären Einheit gekoppelt ist.

11. Vorrichtung nach einem der Ansprüche 8-10,

wobei die Vorrichtung eine induktive Sensoranordnung nach einem der Ansprüche 7 bis 10 umfasst, wobei die Steuerung und die induktive Abtastschaltung (16) von der induktiven Sensoranordnung (50) bereitgestellt werden und die induktive Sensoranordnung funktionsfähig mit der tragbaren handgehaltenen Vorrichtung (14) gekoppelt ist.

12. Vorrichtung nach einem der Ansprüche 8-11, wobei die Vorrichtung eine Signalerfassungs- oder -aufnahmeeinrichtung (40) zum Erfassen von vom Körper zurückgegebenen Signalen basierend auf der Erfassung von Änderungen einer oder mehrerer elektrischer Eigenschaften des Resonanzkreises (30) beinhaltet, und die Vorrichtung Einrichtungen zum Verarbeiten der von den Signalsensoreinrichtungen erfassten Signale und zum Ableiten eines oder mehrerer physiologischer Parameter basierend auf den der erfassten Signalen umfasst.

13. Gerät nach einem der Ansprüche 8 bis 12, wobei das tragbare handgehaltene Vorrichtung (14) ein mobiles Kommunikationsgerät, beispielsweise ein Mobiltelefon, ist.

14. Verfahren zur Steuerung der Funktionalität einer induktiven Sensorschaltung (16) und einer tragbaren handgehaltenen Vorrichtung (14), die elektromagnetische Sendefunktionen aufweist, das Verfahren umfassend ein Implementieren einer Schaltfunktion zum Umschalten zwischen zwei Modi:

einen ersten Modus, in dem die tragbare handgehaltene Vorrichtung gesteuert wird, sodass zumindest ein Teil der elektromagnetischen Emissionen der Vorrichtung deaktiviert wird, und gleichzeitig die induktive Sensorschaltung gesteuert wird, sodass die induktive Sensorschaltung aktiviert wird;

einen zweiten Modus, in dem die tragbare Vorrichtung gesteuert wird, sodass zumindest ein Teil der elektromagnetischen Emissionen der Vorrichtung aktiviert wird, und gleichzeitig die induktive Sensorschaltung gesteuert wird, sodass die Deaktivierung der induktiven Sensorschaltung veranlasst wird,

**dadurch gekennzeichnet, dass** die tragbare handgehaltene Vorrichtung veranlasst wird, eine Stromversorgung von der tragbaren handgehaltenen Vorrichtung zu der induktiven Erfassungsschaltung zu aktivieren, oder alternativ die induktive Erfassungsschaltung zu veranlassen, einen Signalgenerator in der induktiven Erfassungsschaltung zu aktivieren, um die Zufuhr eines Treibersignals zu einer Antenne in der induktiven Erfassungsschaltung auszulösen, und wobei die tragbare handgehaltene Vorrichtung in der zweiten Betriebsart konfiguriert ist, um die Stromversorgung von der tragbaren handgehaltenen Vorrichtung zu der induktiven Erfassungsschaltung zu deaktivieren.

15. Computerprogrammprodukt, umfassend Codeeinrichtungen, die konfiguriert sind, dass sie, wenn sie auf der Steuerung eines der in den Ansprüchen 9-13 beanspruchten Geräte ausgeführt werden, die Steuerung veranlassen, das Verfahren nach Anspruch 14 durchzuführen.

**Revendications**

1. Un contrôleur (12),

le contrôleur comprend une entrée/sortie pour recevoir et transmettre des données lorsque le contrôleur est couplé de manière communicative à un circuit de détection inductif (16) et à un dispositif portable (14), le dispositif portable possédant des fonctions d'émission électromagnétique, et
l'unité de commande fonctionne pour mettre en oeuvre une fonction de commutation pour la commutation entre deux modes:
un premier mode dans lequel le contrôleur est configuré pour communiquer avec le portable l'invention concerne un dispositif portable destiné à provoquer la désactivation d'au moins une partie des émissions électromagnétiques du dispositif et à communiquer simultanément avec le circuit de détection inductif pour provoquer l'activation du circuit de détection inductif; et un second mode dans lequel le contrôleur est configuré pour communiquer avec le l'invention concerne un dispositif portable à main destiné à provoquer l'activation d'au moins une partie des émissions électromagnétiques du dispositif et à provoquer simultanément la désactivation de la détection inductive circuit, **caractérisé en ce que**, dans le premier mode, le contrôleur est configuré pour amener le dispositif portable à activer une alimentation électrique du dispositif portable au circuit de détection inductif ou pour amener le circuit de détection inductif à activer un générateur de signaux dans le circuit de détection inductif afin de déclencher l'alimentation d'un entraînement signal envoyé à une antenne dans le circuit de détection inductif, et dans lequel, dans le second mode, le contrôleur est configuré pour amener le dispositif portable à désactiver ladite alimentation électrique du dispositif portable à main au circuit de détection inductif.

2. Contrôleur (12) selon la revendication 1, dans lequel le premier mode comprend la désactivation d'un ou plusieurs émetteurs électromagnétiques du dispositif portable.

3. Dispositif de commande (12) selon la revendication 1 ou 2, dans lequel le premier mode comprend l'arrêt de l'émission dans une ou plusieurs bandes ou plages de fréquences électromagnétiques, et/ou l'arrêt de toute émission électromagnétique au-dessus d'un seuil défini puissance.

4. Contrôleur (12) selon la revendication 1, configuré en outre pour recevoir un signal de détection inductif entré du circuit de détection inductif et pour dériver du signal un ou plusieurs paramètres physiologiques, par exemple le rythme cardiaque et/ou le rythme respiratoire.

5. Dispositif de commande (12) selon la revendication 1, dans lequel le dispositif de commande est configuré pour mettre en oeuvre une commutation entre les deux modes en réponse à la réception d'une ou plusieurs commandes de commande prédéfinies, et de préférence dans lequel le dispositif de commande est agencé pour recevoir lesdites commandes de commande depuis le dispositif portable à main.

6. Ensemble de détection inductif (50) comprenant: un circuit de détection inductif (16) pour détecter des signaux électromagnétiques renvoyés par un corps en réponse à l'application de signaux d'excitation électromagnétique audit corps, le circuit de détection inductif comprenant un circuit résonateur (30) comprenant une antenne cadre (32), le circuit résonateur pour générer lesdits signaux d'excitation lorsqu'il est commandé par un signal d'excitation,

dispositif de commande (12) selon l'une quelconque des revendications 1 à 5, couplé fonctionnellement au circuit de détection inductif; et
un support (52), le circuit de détection inductif et le contrôleur sont montés sur le support.

7. Ensemble (50) selon la revendication 6, dans lequel le circuit de détection inductif comprend un générateur de signaux pour générer un signal de commande pour commander ladite antenne afin de générer lesdits signaux d'excitation électromagnétiques, et éventuellement, le circuit de détection inductif comprend un moyen de détection ou de détection de signaux destiné à détecter les signaux renvoyés par le corps sur la base de la détection de variations dans une ou plusieurs caractéristiques électriques du circuit résonateur.

8. Un appareil de détection inductive, comprenant:

   L'invention concerne un dispositif portable (14); et
   Contrôleur (12) selon l'une quelconque des revendications 1 à 5.

9. Appareil selon la revendication 8, comprenant en outre un circuit de détection inductif (16) couplé fonctionnellement au contrôleur (12), le circuit de détection inductif étant destiné à détecter des signaux électromagnétiques renvoyés par un corps en réponse à l'application de signaux d'excitation électromagnétiques audit corps, et le circuit de détection inductif comprend un circuit résonateur (30) comprenant une antenne cadre (32), le circuit résonateur générant lesdits signaux d'excitation lorsqu'il est commandé par un signal de commande.

10. Appareil selon la revendication 8 ou 9, dans lequel le circuit de détection inductif (16) est intégré dans le dispositif portable (14), par exemple en tant que partie d'une puce dédiée, ou l'appareil comprend une unité secondaire, le circuit de détection inductif étant intégré dans l'unité secondaire, et le dispositif portable est couplé de manière fonctionnelle à l'unité secondaire.

11. Appareil selon l'une quelconque des revendications 8 à 10, dans lequel l'appareil comprend un ensemble de détection inductif selon l'une quelconque des revendications 7 à 10, le contrôleur et le circuit de détection inductif (16) étant fournis par ledit ensemble de détection inductif (50), l'ensemble de détection inductif est couplé de manière fonctionnelle au dispositif portable (14).

12. Appareil selon l'une quelconque des revendications 8 à 11, dans lequel l'appareil comprend un moyen de détection ou de détection de signal (40) pour détecter des signaux renvoyés par le corps sur la base de la détection de variations dans une ou plusieurs caractéristiques électriques du circuit résonateur (30), l'appareil comprend également des moyens de traitement des signaux détectés par les moyens de détection de signaux et de dérivation d'un ou de plusieurs signaux physiologiques sur la base des signaux détectés paramètres.

13. Appareil selon l'une quelconque des revendications 8 à 12, dans lequel le dispositif portable (14) est un dispositif de communication mobile, par exemple un dispositif de téléphone mobile.

14. L'invention concerne un procédé de commande de la fonctionnalité d'un circuit de détection inductif (16), et un dispositif portable (14) possédant des fonctions d'émission électromagnétique, le procédé consistant à mettre en œuvre une fonction de commutation pour la commutation entre deux modes:

   Un premier mode dans lequel le dispositif portable est commandé pour induire la désactivation d'au moins une partie des émissions électromagnétiques du dispositif, et, simultanément, le circuit de détection inductif est commandé pour induire l'activation du circuit de détection inductif;
   Un second mode dans lequel le dispositif portable est commandé pour provoquer l'activation de ladite au moins une partie des émissions électromagnétiques du dispositif, et, simultanément, le circuit de détection inductif est commandé pour induire la désactivation du circuit de détection inductif, **caractérisé en ce que**, dans le premier mode, le dispositif portable est amené à activer une alimentation électrique du dispositif portable au circuit de détection inductif ou alternativement faire en sorte que le circuit de détection inductive active un générateur de signal dans le circuit de détection inductive pour déclencher l'alimentation d'un signal d'entraînement à une antenne dans le circuit de détection inductive, et dans lequel, dans le second mode, le dispositif portable est amené à désactiver ladite alimentation électrique du dispositif portable au circuit de détection inductif.

15. Produit de programme informatique comprenant un moyen de code configuré, lorsqu'il est exécuté sur le contrôleur de l'un quelconque des appareils selon les revendications 9 à 13, pour amener le contrôleur à exécuter le procédé de la revendication 14.

FIG. 1

FIG. 2

FIG. 3

FIG. 4

FIG. 5

EP 4 125 553 B1

FIG. 6

FIG. 7

FIG. 8

25

FIG. 9

FIG. 10

FIG. 11

FIG. 12

FIG. 13

FIG. 14

**EP 4 125 553 B1**

**Patent documents cited in the description**

- EP 3584896 A

- WO 2018127482 A